# EUROPEAN PATENT APPLICATION

(11) **EP 2 332 509 A1**
(43) Date of publication of application: **15.06.2011**
(21) Application number: 10738541.1
(22) Date of filing: 03.02.2010
(51) Int. Cl.: A61H 33/02, A61H 33/10, A61H 33/14, A61H 35/00, A61K 33/00

(54) **GAS MIST PRESSURE BATH SYSTEM**

(30) Priority: 06.02.2009 JP 2009026766; 06.02.2009 JP 2009026767; 06.02.2009 JP 2009026768; 06.02.2009 JP 2009026769
(71) Applicant: Nakamura, Shoichi, Higashichikuma-gun, Nagano 399-7502 (JP); ACP Japan Co. Ltd., Tokyo 113-0033 (JP)
(72) Inventor: Nakamura, Shoichi, Nagano 399-7502 (JP)
(74) Representative: Gill, David Alan
(86) International application number: PCT/JP2010/051492
(87) International publication number: WO 2010/090210

(57) **Abstract**

The invention is to provide a gas mist pressure bath system, which is possible to control the amount of gas and liquid, pressure and others, and cause a gas mist to be absorbed through a skin and mucous membrane of a human living body under an optimum condition. The present system comprises a gas supply means 11, a gas mist supply means 31 for generating and supplying the gas mist by pulverizing and dissolving the gas and the liquid supplied from the gas supply means 11, and a living-body cover member 41 for covering the skin and mucous membrane of the living body and forming a space of sealing inside the gas mist supplied from the gas mist supply means 31, wherein the gas mist supply means 31 comprises a liquid storing part 32B, a gas mist storing part 32A, a nozzle part 33 of jetting gas from its front end, a suction pipe 36A of sucking up the liquid until the nozzle front end, and a baffle member 35 provided opposite the nozzle front end, and that the gas jetted from the nozzle part 33 causes the liquid sucked up by the suction pipe 36A until the nozzle front end to collide with the baffle member 35 and turn out the gas mist by pulverizing and dissolving.

## Description

### Technical Field

The present invention relates to a gas mist pressure bath system, in which a mist (called as "gas mist" hereafter) is prepared by pulverizing and dissolving carbon dioxide or oxygen, or a mixed gas (called as "gas" hereafter) of carbon dioxide and oxygen, and liquid, and the thus prepared gas mist is directly contacted to a skin and mucous membrane of a human living body at pressure of not less than a predetermined value, thereby to improve a gas absorption efficiency into skin and mucous membrane.

### Background Art

It has conventionally been known that carbon dioxide (carbonic acid anhydride: CO₂, called as "carbon dioxide" hereafter) has both properties of being not only soluble in water (water-soluble) but also soluble in fat (fat-soluble) and, therefore, by only contacting the skin and mucous membrane of the living body which is like mixed with water and fat, carbon dioxide penetrates under a subcutaneous layer and expands blood vessels around parts of penetrated carbon dioxide, and it works to improve a blood circulation. Owing to this action of accelerating the blood circulation, it displays various physiological effects such as dropping of blood pressure, improving of metabolism or accelerating to remove pain substance or waste product. Further, it has also anti-inflammation and anti-bacterial. Therefore, carbon dioxide has recently been given attentions also from viewpoints of improving health or beauty other than the purpose of medical cares.

Carbon dioxide in the tissue of the living body works to release oxygen carried in combination with hemoglobin in a red blood cell. Around parts at a high density of carbon dioxide, the red blood cell releases more oxygen. Thus, supply of oxygen to cells by the red blood cell is mainly controlled by carbon dioxide. In short, being without carbon dioxide, hemoglobin remains as combined with oxygen and the cell becomes unable to receive oxygen. As is seen, carbon dioxide seems to be a waste product resulted from action of the cell, however, it plays in fact very important roles in the human living body.

Further, in recent times, oxygen of high density has also widely been known as effective in activity of metabolism, accelerating the blood circulation, fatigue recovery, or stability of blood pressure. Other than them, oxygen has disinfection or sterilization by oxidative effect.

As a prior art for causing carbon dioxide to be absorbed into the living body, a most broadly used technique is (1) a bathing agent issuing carbon dioxide in water. Throwing this bathing agent into hot water in a bathtub, it generates carbon dioxide by reacting carbonate and acid contained in the bathing agent, and dissolves it in hot water. Carbon dioxide dissolved in hot water contacts the skin of a bathing person and penetrates his subcutaneous layer to display physiological effects as above mentioned.

As the prior art for causing more carbon dioxide to contact the living body, (2) a carbon dioxide bathing device is known. This emits and disperses carbon dioxide in hot water and dissolves it at high density. When bathing in hot water dissolving carbon dioxide, the skin directly contacts it like the above mentioned bathing agent.

A blood circulation accelerating device (for example, Patent Document 1) has now been disclosed, which (3) attaches a cover of forming a sealed space together with the surface of a human living body to the human living body on its surface, and introduces carbon dioxide into the sealed space from a carbon dioxide supply means for carrying out a carbon dioxide bath.

A carbon dioxide pressure bath device which is equipped with at least (4) the carbon dioxide supply means, a pressurizing means, and a covering material for covering the living body' s skin and causing carbon dioxide to contact the skin at pressure of not less than predetermined value, has been proposed by an inventor of the present invention.

As the prior art for causing oxygen to be absorbed into the living body, (5) a high density oxygen bathing device has been known. Being similar to the carbon dioxide bathing device, this emits and disperses carbon dioxide in hot water, in which taking a bath, oxygen is caused to directly contact the skin.

### Citation List

### Patent Documents

Patent Document 1: Japanese Patent Application Publication No. 07-171189

### Summary of Invention

### Problems to be Solved by the Invention

However, each of the above prior arts (1), (2) and (5) dissolves carbon dioxide or oxygen in hot water when taking the bath, and causes carbon dioxide or oxygen to be absorbed into the skin of the living body. Accordingly, they were involved with difficult points of using only when taking the bath. In addition, since carbon dioxide is easily dissolved in water, and even if much consuming it for dissolving in hot water, an absorption rate into the skin is never much high.

On the other hand, since the above prior arts (3) and (4) cause carbon dioxide to directly contact the living body and if comparing with the prior arts (1) and (2), effects are high and efficiency is good. But these have not optimized to control the amount or pressure of carbon dioxide, oxygen and the mist to be introduced into the shielding member (cover).

In view of the above mentioned problems, it is an object of the invention to provide a gas mist pressure bath system which is possible to control the amount, pressure and others of gas and liquid, and cause the gas mist to be absorbed through the skin and mucous membrane of the human living body under an optimum condition.

### Means for Solving the Problems

For solving the above mentioned problems, the present invention is to provide a gas mist pressure bath system, in which a mist (called as "gas mist" hereafter) is prepared at a density of not less than a predetermined value by pulverizing and dissolving carbon dioxide or oxygen, or a mixed gas (called as "gas" hereafter) of carbon dioxide and oxygen, and liquid, and the thus prepared gas mist is contacted to the skin and mucous membrane of the living body. The present system comprises a gas supply means, a gas mist supply means for generating and supplying the gas mist by pulverizing and dissolving the gas and the liquid supplied from the gas supply means, and a living-body cover member for covering the skin and mucous membrane of the living body and forming a space of sealing inside the gas mist supplied from the gas mist supply means, wherein the gas mist supply means comprises a liquid storage of storing liquid, a gas mist storage of storing a gas mist, a nozzle part of jetting gas from its front end, a suction pipe of sucking up the liquid stored in the liquid storage until the nozzle front end, and a baffle member provided opposite the nozzle front end, and is characterized in that the gas jetted from the nozzle part causes the liquid sucked up by the suction pipe until the nozzle front end to collide with the baffle member and turn out the gas mist by pulverizing and dissolving.

By the way, the invention refers it as "pulverizing and dissolving" to pulverize the liquid into fine liquid drops, and cause to contact and mix with gas (carbon dioxide, or oxygen, or a mixed gas of carbon dioxide and oxygen).

Herein, desirably, the above mentioned gas mist pressure bath system of the invention is further provided with a sensor for measuring supplying conditions of the gas, liquid and gas mist, and control means for controlling supplies of the gas, liquid and gas mist based on the measuring values of the sensors.

In addition, the above gas mist pressure bath system is desirably further provided with a pressurizing means for pressurizing the living-body cover member.

The control means may supply the gas mist intermittently into the living-body cover member to perform interval pressurization (pulse pressurization) thereon. Otherwise, the pressurizing means may pressurize the living-body cover member intermittently to perform the interval pressurization (pulse pressurization) thereon.

Further, the gas mist pressure bath system of the present invention is desirably provided with a liquid supply means of supplying liquid to the above mentioned liquid storage. A liquid pressurizing means may be furnished for pressurizing the liquid from the liquid supply means and supplying it to the gas mist supply means.

It is optimum that the above mentioned liquid is any one or plural combination of water, ionic water, physiological salt solution, ozone water, purified water or sterilized water. This liquid desirably further contains any one or plural combination of menthol, vitamin E, vitamin C derivative, retinol, anesthetic, cyclodextrin, photocatalyst, complex of photocatalyst and apatite, hyaluronic acid, coenzyme Q10, seed oil, propolith, ethanol, gluconic acid chlorohexizine, amphoteric surface active agent, benzalkonium chloride, alkyldiamino ether glycin acetate, sodium hypochlorite, acetyl hydroperoxide, sodium sesquicarbonate, silica, povidone-iodine, sodium hydrogen carbonate, high density carbonate spring, anti-allergic agent, anti-inflammatory agent, anti-febrile, anti-fungus agent, anti-influenza virus, carcinostatic substance, anti-hyper tensive agent, cosmetic agent, or trichogen.

Preferably, the liquid is supplied into the gas mist supply means under a condition of being heated. Sizes of the gas mist supplied from the gas mist supply means into the living-body cover member are suitably not more than 10 µm.

The control means preferably holds pressure at 1.02 to 2.5 air pressure in the living-body cover member when taking the gas mist bath.

There may be provided an electric charge supply means for supplying charge to the mist from the gas mist supply means. At this time, the charge is preferably minus.

Desirably, the gas mist supply means has a gas mist supply pipe for supplying the gas mist into the living-body cover member, and this gas mist supply pipe has a filter for removing liquid drops attached to a pipe inside. Further, a whole or one part of the gas mist supply pipe is suitably composed of a cornice shaped pipe, and this gas mist supply pipe is provided with a check valve.

In addition, the gas mist supply portion of the living-body cover member is also provided at its supply portion with the check valve.

Further, the gas mist storage is shaped in dome of convex having a curved face toward an upper portion and is formed with a gas mist exhaust portion at the dome shaped top. The gas mist storage has desirably one or plurality of pored plates for refining the gas mist.

The control means stops the gas from the gas supply means when the pressurizing value within the living-body cover member is higher than the predetermined value.

### Advantageous Effect of the Invention

According to the gas mist pressure bath system of the invention, since it is possible to control the amount and pressure of the gas mist in the living-body pressure bath cover by the control device, the gas mist bath can be always taken under the best condition.

Being able to generate the gas mist with a very simple structure, a low cost of the device can be realized.

Further, pressurization into the living-body pressure bath cover is easy, and a skin-pass breath of the gas can be carried out more efficiently.

### Brief Description of Drawings

[FIG. 1] A generally schematic view of the gas mist pressure bath system depending on a first embodiment of the invention;
[FIG. 2] A cross sectional view of the mist generating part in the gas mist supply means of the gas mist pressure bath system of the invention;
[FIG. 3] A perspective view, partially in section, of the mist generating part in the gas mist supply device of the gas mist pressure bath system of the invention;
[FIG. 4] A typical view showing one example of the gas mist supply pipe used to the gas mist pressure bath system of the invention;
[FIG. 5] A cross sectional view, partially in section, showing another example of the gas mist supply device of the gas mist pressure bath system of the invention;
[FIG. 6] A typical view showing one example of the plates to be placed within the gas mist supply device of the gas mist pressure bath system of the invention;
[FIG. 7] A typical view showing a configuration example (No.1) of the living-body pressure bath cover of the gas mist pressure bath system depending on the first embodiment of the invention;
[FIG. 8] A typical view showing a configuration example (No.2) of the living-body pressure bath covers of the gas mist pressure bath system depending on the first embodiment of the invention;
[FIG. 9] A typical view showing a configuration example (No.3) of the living-body pressure bath cover of the gas mist pressure bath system depending on the first embodiment of the invention;
[FIG. 10] A generally schematic view of the gas mist pressure bath system depending on a second embodiment of the invention;
[FIG. 11] A typical view showing a configuration example (No.1) of the living-body pressure bath cover of the gas mist pressure bath system depending on the second embodiment of the invention;
[FIG. 12] A typical view showing a configuration example (No.2) of the living-body pressure bath cover of the gas mist pressure bath system depending on the second embodiment of the invention;
[FIG. 13] A generally schematic view of the gas mist pressure bath system depending on a third embodiment of the invention, and
[FIG. 14] A generally schematic view of the gas mist pressure bath system depending on a fourth embodiment of the invention.

### Description of Embodiments

In the following description, explanations will be made to embodiments of this invention, referring to the attached drawings.

### [First Embodiment]

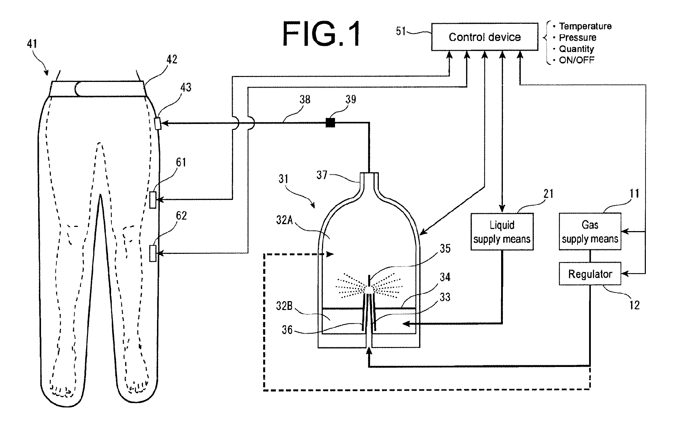

FIG. 1 is the generally schematic view of the gas mist pressure bath system depending on the first embodiment of the invention. As shown in this view, the gas mist pressure bath system of this embodiment comprises the gas supply means 11, the liquid supply means 21, the gas mist supply device 31 having the storage 32 composed of a gas mist storing part 32A and a liquid storing part 32B, generating the gas mist by pulverizing and dissolving the liquid and the gas (carbon dioxide, or oxygen, or a mixed gas of carbon dioxide and oxygen), and supplying it under pressurization, the living-body pressure bath cover 41 of forming a space for sealing the supplied gas mist, and the control device 51 for generating and controlling to supply the gas mist.

The gas supply means 11 is connected to a nozzle 33 of the gas mist supply device 31, and discharges gas from the front end of the nozzle 33 into the gas mist storing part 32A, provided that if the gas mist is enough supplied in the living-body pressure bath cover 41, the gas is not supplied into the stored liquid, but directly supplied into the gas mist storing part 32A as shown with a dotted line in FIG. 1. As the gas supply means 11, to use a gas bomb is optimum. The gas supply means 11 is provided with a regulator 12 for adjusting gas pressure. Omitting illustration, the gas supply means 11 may be disposed with a heater for heating the gas or a thermometer for controlling temperatures.

The liquid supply means 21 is composed of such as a pump and supplies water to the gas mist supply device 31. As the liquid, it is suitable to use water, ionic water, physiological salt solution, ozone water, purified water or sterilized water. Further, these liquids may contain medicines useful to user's diseases or symptoms. For the medicines, enumerated are, for example, anti-allergic agent, anti-inflammatory agent, anti-febrile, anti-fungus agent, anti-influenza virus, carcinostatic substance, anti-hypertensive agent, cosmetic agent, or trichogen. Further, these liquids are further possible to generate synergistic effects by coupling with a gas physiological action with single or plurality of menthol having a cooling action; vitamin E accelerating circulation of the blood; vitamin C derivative easily to be absorbed to a skin tissue and having a skin beautifying effect; retinol normalizing a skin heratinizing action and protecting the mucous membrane; anesthetic moderating irritation to the mucous membrane; cyclodextrin removing odor; photocatalysis or a complex of photocatalysis and apatite having disinfection and anti-phlogistic; hyaluronic acid having excellent water holding capacity and a skin moisture retention effect; coenzyme Q10 activating cells and heightening immunization; a seed oil containing anti-oxidation and much nutrient; or propolith having anti-oxidation, anti-fungus, anti-inflummatory agent, pain-killing, anesthetic, and immunity. Otherwise, the liquids may be added with ethanol, gluconic acid chlorohexizine, amphoteric surface active agent, benzalkonium chloride, alkyldiamino ether glycin acetate, sodium hypochlorite, acetyl hydroperoxide, sodium sesquicarbonate, silica, povidone-iodine, sodium hydrogen carbonate. In addition, high density carbonate spring may be added (as examples organic components, sulfate, carbonate, sodium dichloroisocyanurate) having main components of carbonate and organic acid.

By the way, in the liquid supply means 21, it is desirable to dispose a heater (not shown) heating the liquid (for example, heating to a hot water of around 40°C) or a thermometer (not shown) controlling temperature.

The gas mist supply device 31 has the storage 32 (the gas mist storing part 32A and the liquid storing part 32B), the nozzle 33 of discharging the gas supplied from the gas supply means 11 out of the front end, the suction pipe 36A of sucking up the liquid stored in the liquid storing part 32B until the front end of the nozzle 33, and a baffle member (collision member) 35 provided opposite the nozzle front end. Although omitting illustration, there are provided respectively a gas supply port for guiding the gas from the gas supply means 11 to the gas mist storing part 32A and a liquid supply port for guiding the liquid from liquid supply means 21 to the storing part 32B.

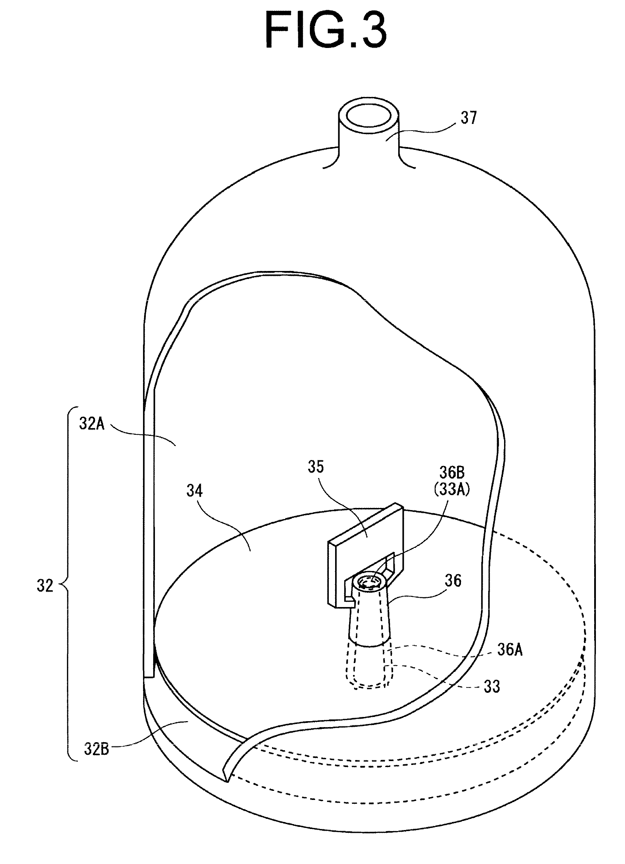

The storage 32 is, as shown in FIG. 3, is divided into the gas mist storing part 32A and the liquid storing part 32B by a shielding plate 34. The above side (the opening side 33A of the nozzle front end) of the shielding plate 34 is the gas mist storing part 32A of storing the generated gas, while the under side (the bottom side of the storage 32) is the liquid storing part 32B of storing the liquid.

At the bottom center of the storage 32, a nozzle 33 is provided. This nozzle 33 is formed to be substantially conic toward an upper part from the bottom side. Its basic end is connected to the gas supply means 11 outside of the device, while its front end projects toward the side of the gas mist storing part 32A, and it is possible to discharge gas from the gas mist storing part 32A. The nozzle 33 is connected at its basic end to the gas supply means 11 directly or via a tube, and desirably, a connection portion is composed with such as a connector of one-touch.

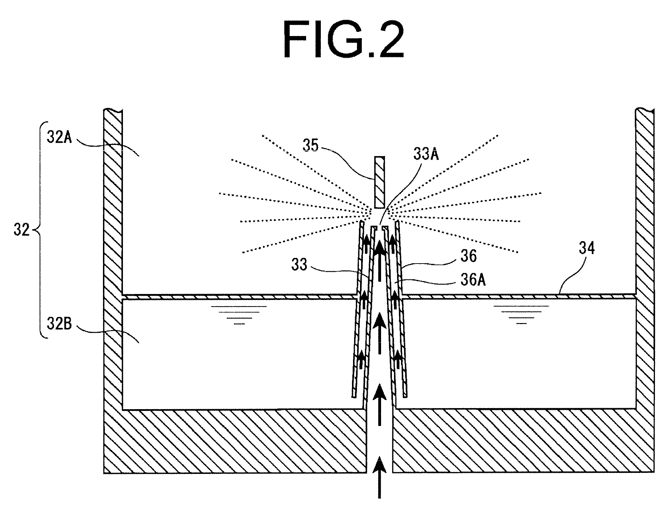

The suction pipe 36A is formed between the nozzle 33 and a suction pipe-forming member 36 being substantially conic and larger in size than the nozzle 33. That is, as shown in FIG. 2, by covering the suction pipe-forming member 36 over the nozzle 33, the suction pipe 36A is defined between the outer circumference of the nozzle 33 and the suction pipe-forming member 36. Further, between the basic end of the suction pipe-forming member 36 and the bottom of the liquid storing part 32B, space is defined from which the liquid stored in the liquid storing part 32B is sucked by the suction pipe 36A. The front end 36B of the suction pipe-forming member 36 opens nearly a front end opening 33A, and this is so structured that the liquid sucked up along the suction pipe 36A collides with the gas flow discharged from the nozzle 33.

The baffle 35 is a member which is positioned opposite the front end opening 33A of the nozzle 33 and the front end 36B of the suction pipe-forming member 36, and herein this is connected to the suction pipe-forming member 36. The baffle 35 may be structured to connect to the shielding plate 34 or the storage 32. By the way, the suction pipe-forming member 36 is connected to the shielding plate 34 at a central portion in the vertical direction. Besides, the outer circumference of the shielding plate 34 is connected to the inside of the storage 32. Thus, desirably, the whole of the gas mist supply device 31 is formed as one-body.

The shielding plate 34 operates to force liquid upward of the suction pipe-forming member 36 by keeping pressure higher within the liquid storing 32B than that of the gas mist storing part 32A. Therefore, the shielding plate 34 may be secured at a determined position of an inside wall of the liquid storing part 32B, but may be structured to be movable vertically in response to the level of the liquid surface in the liquid storing part 32B. Further, depending on magnification of gas pressure discharged from front end opening 33A, the shielding plate 34 may be absent.

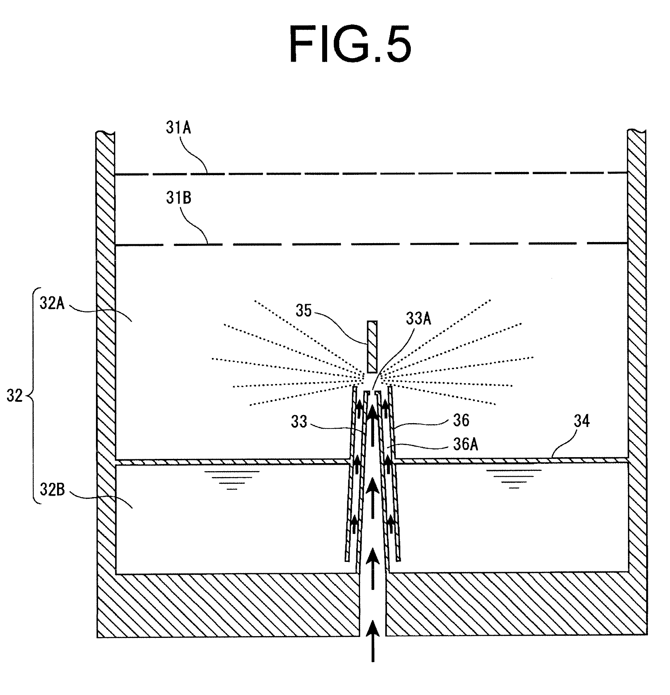

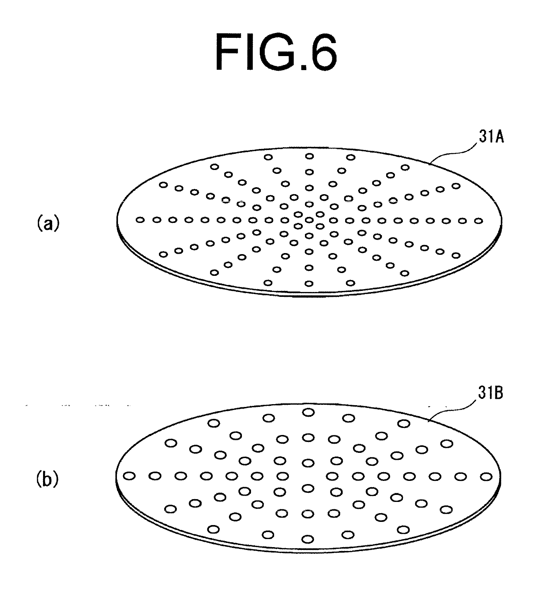

As shown in FIG. 5, above the nozzle 33 in the gas mist storing part 32A, one sheet or plural sheets of plates 31A, 31B (in FIG. 5, as the example, two sheets) are furnished. FIG. 6 shows examples of the plates 31A, 31B. The plates 31A, 31B are formed with plural pores, and the generated gas mist is further refined by passing through the pores. Then, with respect to the upper plate 31A and the lower plate 31B, it is preferable that the diameter of the pore in the upper plate 31A is smaller than that of the lower plate 31B.

For generating the gas mist by the above mentioned gas mist supply device 31, the gas from the gas supply means 11 is supplied to the nozzle 33 under a condition of having stored the liquid in the liquid storing part 32B. Then, since the nozzle 33 is reduced in diameter toward the front end, the gas increases flowing speed and discharged. The liquid is sucked upward in the suction pipe 36A owing to negative pressure caused by air current, and is blown up by the air current near the front end opening 33A of the nozzle 33 and collides with the lower end of the baffle 35. By this collision, the liquid is pulverized, mixed with gas and dissolved. Sizes of the mist generated at this time are desirably fine, and concretely, being less than 10 µm is optimum.

The generated gas mist spreads over inside of the storing part 32A and is discharged from the gas mist exhaust portion 37 following a gas convection. Herein, it is desirable that the storing part 32A is shaped in convex dome toward an upper portion having a curved face as shown in FIG. 1. The gas mist exhaust portion 37 is provided at a head top of the dome shape. By shaping such a form, it is possible that the gas mist is more stored, while preventing that the mist contacts the top portion of the inside wall of the storing part 32A and reverts to the liquid.

The gas mist discharged from the gas mist exhaust portion 37 is supplied into the living-body pressure bath cover 41 via the gas mist supply pipe 38. The gas mist supply pipe 38 is connected to a supply portion 43 of the living-body pressure bath cover 41, and is desirably furnished with a liquid drop removing filter 39 for removing excessive liquid drop attached to the pipe inside. Although not illustrating, the gas mist supply pipe 38 is provided inside with a check valve for checking back-flow of gas mist and gas.

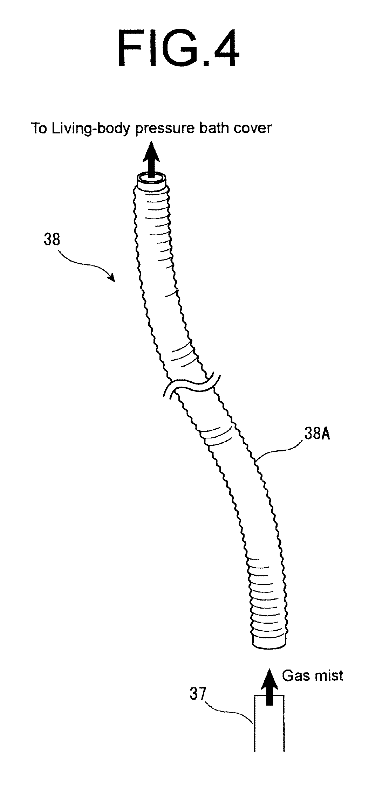

Further, as shown in FIG. 4, preferably, the gas mist supply pipe 38 is overall or partially composed of a soft cornice shaped pipe 38A of a large diameter. If composing with such a corniced pipe 38A, the gas mist supply pipe 38 is freely bent and may be expanded so that the system user is not restricted in his action. Even if the gas mist flowing in the gas mist supply pipe 38 becomes gradually liquefied, the liquid can be removed through concaves and convexes of the cornice.

By the way, in the above described structure, the liquid in the liquid storing part 32B is supplied from the liquid supply means 21, but it is sufficient to provide such a structure, omitting the liquid supply means 21, in advance containing a liquid (liquid medicine). In such a case, preparing the gas mist supply device 31 having in advance contained the liquid and sealed, it is, at using, connected with the gas supply means 11 and the living-body pressure bath cover 41 for taking a gas mist pressure bath. After using, only the gas mist supply device 31 is taken away and disused. Being structured as disposable, the gas mist pressure bath can be taken hygienically and conveniently.

The living-body pressure bath cover 41 may form a space for covering the skin and mucous membrane of the living body (herein, as the example, a lower extremity) and sealing the gas mist and the gas inside. The living-body pressure bath cover 41 is composed of a pressure resistant, non-air permeable and non-moisture permeable material, for example, preferably, the natural rubber, silicone rubber, polyethylene, polypropylene, polyvinylidene, polystylene, polyvinylacetate, polyvinyl chloride, polyamide resin, polytetrafluoroethylene. The living-body pressure bath cover 41 has a supply portion 43 for introducing the gas mist and the gas inside. The supply portion 43 is inside provided with a check valve for checking back flow of the gas mist and the gas. The living-body pressure bath cover 41 may be provided with an opening or a valve for exhausting the gas and the gas mist. The pressure control may be carried out manually, but as later mentioned, desirably automatically by a control device 51 together with supply control of the gas and the gas mist on the basis of measuring values of the manometer 61. A safety valve (recess valve) may be provided for automatically opening a valve when the inside of the living-body pressure bath cover 41 becomes more than a constant pressure.

The living-body pressure bath cover 41 is inside installed with a manometer 61 for measuring an inside pressure. The control device 51 controls supply of the gas mist and the gas on the basis of measuring values of the manometer 61 for maintaining a pressure value within the living-body pressure bath cover 41 to be more than 1 air pressure (more preferably, around 1.02 to 2.5 air pressure). For example, the control device 51 controls or stops the supply of the gas from the gas supply means 11, or exhausts the gas or the gas mist from the living-body pressure bath cover 41. Further, the living-body pressure bath cover 41 is inside installed with a thermometer 62 for measuring an inside temperature within the living-body pressure bath cover 41. The control device 51 performs on-off of a heater installed in the liquid supply means 21 on the basis of measuring values of a manometer 62 for maintaining a predetermined temperature (for example, around 38°C) bringing about warm bath effects within the living-body pressure bath cover 41.

The living-body pressure bath cover 41 has, around its opening, a stopper 42 for attaching to and detaching from the living body (herein, as the example, the lower extremity) and stopping leakage of the gas mist and the gas. The stopper 42 is suitably composed of, e.g., a face stretching fastener, or may have a sole string or rubber or their combination. For heightening a sealing property in the living-body pressure bath cover 41, the inside (that of the stopper 42) may have a material attaching to the user' s skin. The adhesive material is preferably, e.g., a visco-elastic gel of polyurethane or silicone rubber. Further, this adhesive material is detachably used and exchangeable each time or if viscosity becomes weak.

The control device 51 is composed of a computer having CPU, memory and display. This device performs various kinds of controls such as pressure control or on-off switch of the gas from the gas supply means 11, supply pressure or temperature of the liquid from the liquid supply means 21, on-off switch of the gas mist, on-off switch of the gas mist in order to perform the gas mist pressure bath under an optimum condition. In particular, it is preferable to compose a structure when the pressure value becomes a predetermined value in the living-body pressure bath cover 41, supplying of the gas from the gas supply means 11 is stopped by the control device 51.

For carrying out the gas mist pressure bath by using the gas mist pressure bath system of the present embodiment, the living-body pressure bath cover 41 is secured to the living body (herein, as the example, the lower extremity) and closed. In the liquid storing part 32B of the gas mist supply device 31, a liquid of a predetermined amount is in advance poured, and next, the gas is supplied from the gas supply means 11 to the nozzle, thereby to generate the gas mist. Then, the control device 51 controls the supplying pressure, amount, temperature and others of the liquid and the gas. The generated gas mist is supplied from the supply portion 43 into the living-body pressure bath cover 41. When the mist is enough filled in the living-body pressure bath cover 41, the gas is not supplied into the nozzle 33, but directly supplied into the gas mist storing part 32A. When the inside of the living-body pressure bath cover 41 becomes an optimum pressurized and heated condition (around 1.02 to 2.5 air pressure and around 38°C) in view of the measuring values of the manometer 61 and the thermometer 62, the control device 51 once stops supply of the gas mist or the gas and under this condition the gas mist pressure bath is carried out.

The above mentioned explanation has been made with the example of the lower extremities of the living body, and the invention is applicable to various parts. Then, the optimum gas mist pressure bath is performed using the living-body pressure bath cover 41 meeting object parts of the living body.

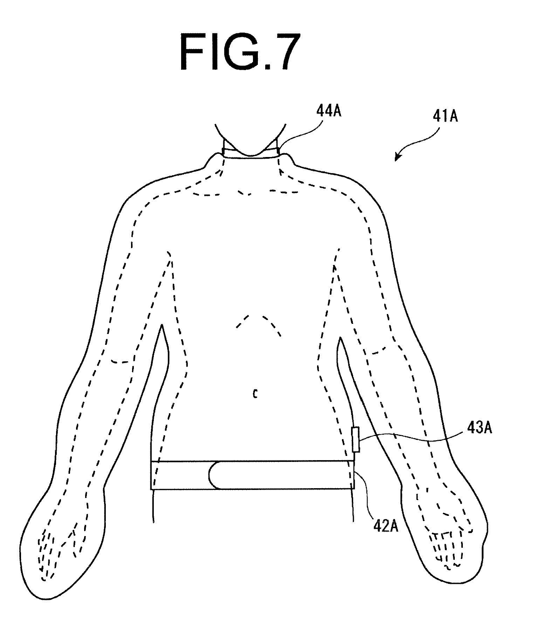

FIG.s 7 to 9 show the various shaped examples of the living-body pressure bath covers 41. At first, FIG. 7 shows the schematic view of the living-body pressure bath cover 41A for the upper half of the body. The living-body pressure bath cover 41A has a shape for wrapping the whole of the upper half of the body, and has a stopper 42A for attaching to and detaching from the living body and stopping leakage of the gas mist and the gas. A similar stopper 44A is formed around the opening of a neck. 43A designates a supply portion for introducing the gas mist and the gas inside.

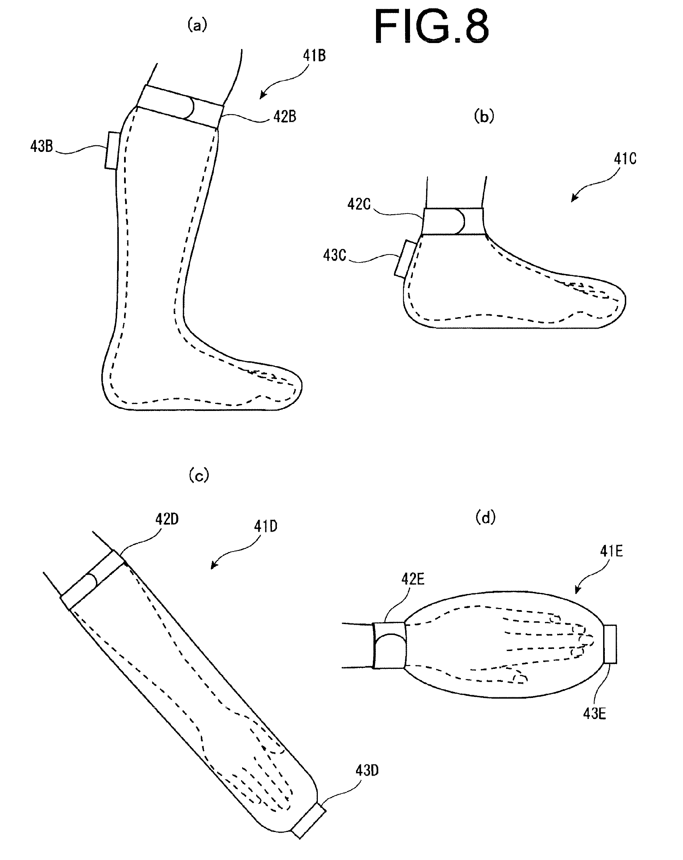

FIG. 8 shows the various shaped examples of the living-body pressure bath covers 41 for covering further limited parts of the living body. FIG. 8 (a) is a living-body pressure bath cover 41B for one-side lower extremity (lower part under a knee) of the living body. The living-body pressure bath cover 41B has a stopper 42B at its opening part and a supply portion 43B for introducing the gas mist and the gas inside. FIG. 8(b) is a living-body pressure bath cover 41C for a foot of the living body. The living-body pressure bath cover 41C has a stopper 42C at its opening part and a supply portion 43C for introducing inside the gas mist and the gas. FIG. 8 (c) is a living-body pressure bath cover 41D for a forearm of the living body. The living-body pressure bath cover 41D has a stopper 42D and a supply portion 43D for introducing inside the gas mist and the gas. FIG. 8(d) is a living-body pressure bath cover 41E for a hand of the living body. The living-body pressure bath cover 41E has a stopper 42E and a supply portion 43E for introducing inside the gas mist and the gas.

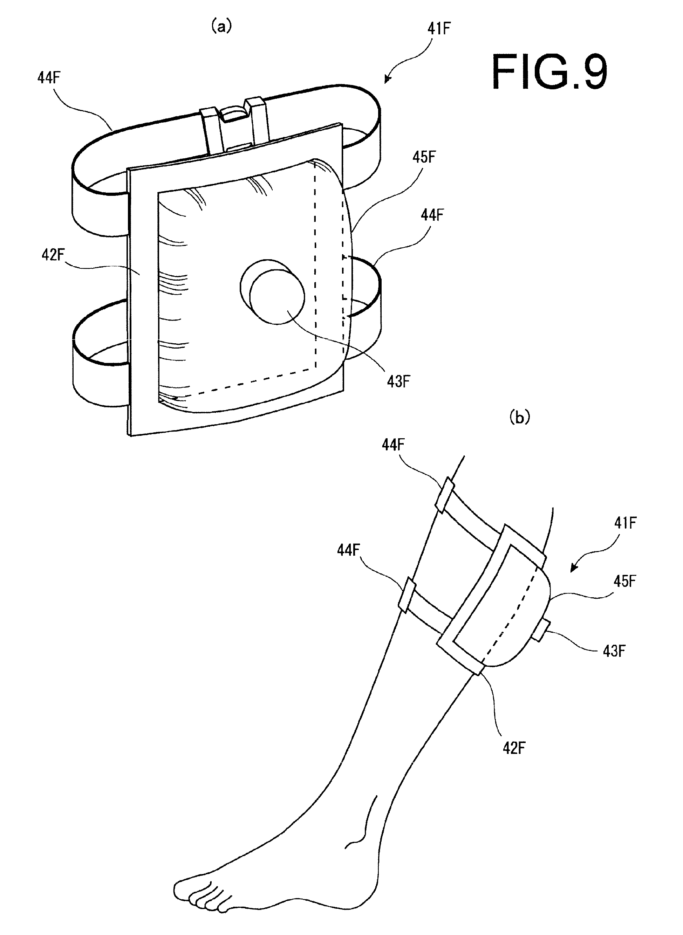

Further, FIG. 9 shows a patch shaped living-body pressure bath cover 41F. FIG. 9(a) is a view showing an outline of the patch shaped living-body pressure bath cover 41F. FIG. 9 (b) is a view showing an external appearance when attaching the patch shaped living-body pressure bath cover 41F to the living body (herein, lower extremity of the living body). The living-body pressure bath cover 41F is composed of a cover part 45F for covering the skin and mucous membrane of the living body, a stopper 42F provided at the margin of the cover part 45F and directly attached to the skin and mucous membrane of the living body, a supply portion 43F for supplying the gas mist and the gas into a space defined by the cover part 45F and the stopper 42F, and fasteners 44F made of belts or strings for fastening the cover part 45F to the living body.

In regard to the living-body pressure bath covers 41, other than the examples shown in FIG.s 7 to 9, various shapes may be assumed. In sum, if forming the space for sealing the gas mist and the gas inside, any shape is sufficient. An exhaust portion may be formed for exhausting the gas mist and the gas from the inside of the living-body pressure bath covers 41. In addition, the invention may be applied not only to the human living body but to animals.

In addition, since pressurization in the gas mist pressure bath heightens the effects by pressurizing in pulsing at predetermined interval, the control device 51 may supply the gas mist into the living body pressure bath cover 41 intermittently at fixed rhythm. As to the pressurizing interval at such a case, if synchronizing with pulsations, the effects are more heightened.

### [Second Embodiment]

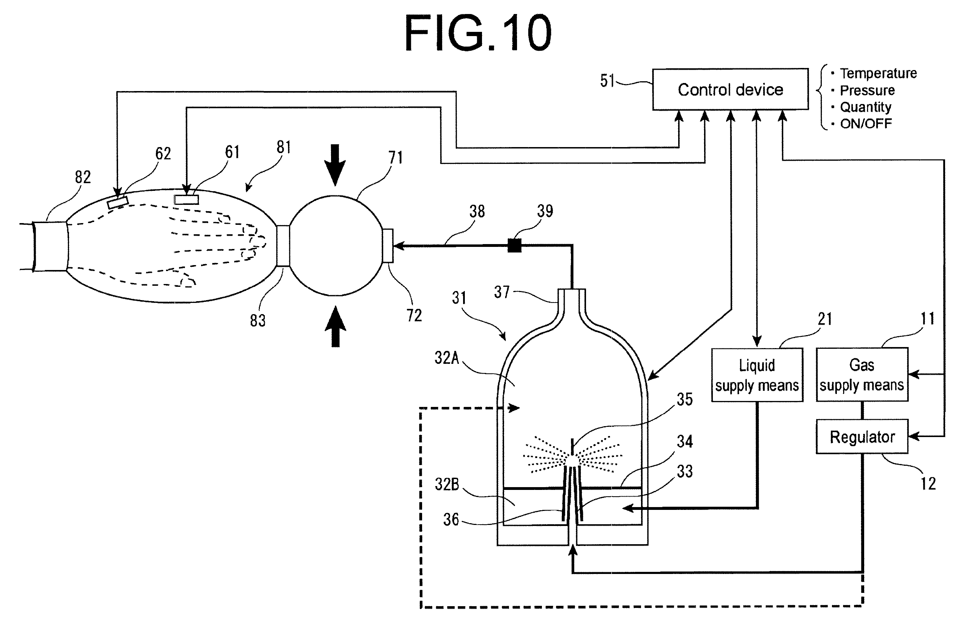

FIG. 10 is the whole schematic view of the gas mist pressure bath system depending on the second embodiment of this invention. This embodiment will explain the gas mist pressure bath system further having a pressurizing means for simplifying pressurization within the living-body pressure bath cover. As to the same parts as those of the first embodiment shown in FIG. 1, the same numerals will be given, and detailed explanation will be omitted.

As shown in FIG. 10, the gas mist pressure bath system of this embodiment has a living-body pressure bath cover 81 forming a space into which the gas mist and the gas are sealed and a pressurizing part (gas storage) 71 connecting the living-body pressure bath cover 81 and pressurizing therein.

The living-body pressure bath cover 81 has almost the same structure of the living-body pressure bath cover 41 of the first embodiment, and has a stopper 82 and a gas mist and gas supply portion 83, provided herein that the supply portion 83 is connected to the pressurizing part 71. By the way, the example hereof illustrates the living-body pressure bath cover 81 of a shape for covering a hand of the human living body.

The pressurizing part 71 is the hollow gas storage connecting the living-body pressure bath cover 81 and pressurizing therein. The pressurizing part 71 is connected to the supply portion 83 of the living-body pressure bath cover 81 and has also a supply portion 72 of itself from which the gas mist or the gas are supplied therein. The supply portion 72 of the pressurizing part 71 is also provided with the check valve for checking back flow of the gas mist and the gas. After storing the gas mist or the gas in the pressurizing part 71, if pressurizing as crushing the pressurizing part 71 as showing with arrows, since the gas mist or the gas in the pressurizing part 71 are exhausted as escaping into the living-body pressure bath cover 81, the inside of the living-body pressure bath cover 81 can be pressurized.

The pressurizing part 71 may be structured as pressing manually, and mechanically by controlling the control means 51 using a driving device. As mentioned above, pressurization in the gas mist pressure bath heightens effects by performing interval pressurization in pulse, and so the pressurizing part 71 may be pressed intermittently. The pressurizing interval heightens effects by synchronizing with pulsation of pulse.

For carrying out the gas mist pressure bath by using the gas mist pressure bath system of the present embodiment, the living-body pressure bath cover 81 is secured to the living body (herein, as the example, the living-body's hand) and closed. In the liquid storing part 32B of the gas mist supply device 31, a liquid of a predetermined amount is in advance poured, and next, the gas is supplied from the gas supply means 11 to the nozzle 33, thereby to generate the gas mist. Then, the control device 51 controls the supplying pressure, amount, temperature and others of the liquid and the gas. The generated gas mist is supplied from the supply portion 83 via the pressurizing part 71 into the living-body pressure bath cover 81. When the mist is enough filled in the living-body pressure bath cover 81, the gas is not supplied into the nozzle 33, but directly supplied into the gas mist storing part 32A. The control device 51 so controls that the inside of the living-body pressure bath cover 81 is to be at an optimum temperature (for example, around 38°C) from the measuring values of the thermometer 62. When the gas mist or the gas of the optimum amount is stored in the living-body pressure bath cover 81 and the pressurizing part 71, the pressurizing part 71 is pressurized as crushed. Thereby, the gas mist or the gas in the pressurizing part 71 are exhausted into the living-body pressure bath cover 81, and the inside of the living-body pressure bath cover 81 is pressurized moderately (around 1.02 to 2.5 air pressure) and the gas mist pressure bath is carried out.

As having mentioned in the first embodiment, since the living-body pressure bath cover 81 is applied to various parts of the living body, many shapes may be used, provided in this embodiment that shapes (size) must be easily pressurized by the pressurizing part 71. This substantially depends on the dimension of the pressurizing part 71. Actually, so far as pressurizing means are any one, the pressurizing part 71 is desirably compact as not demanding large spaces, and accordingly, the living-body pressure bath cover is also desirably applied to comparatively compact objects (covering limited parts of the living body).

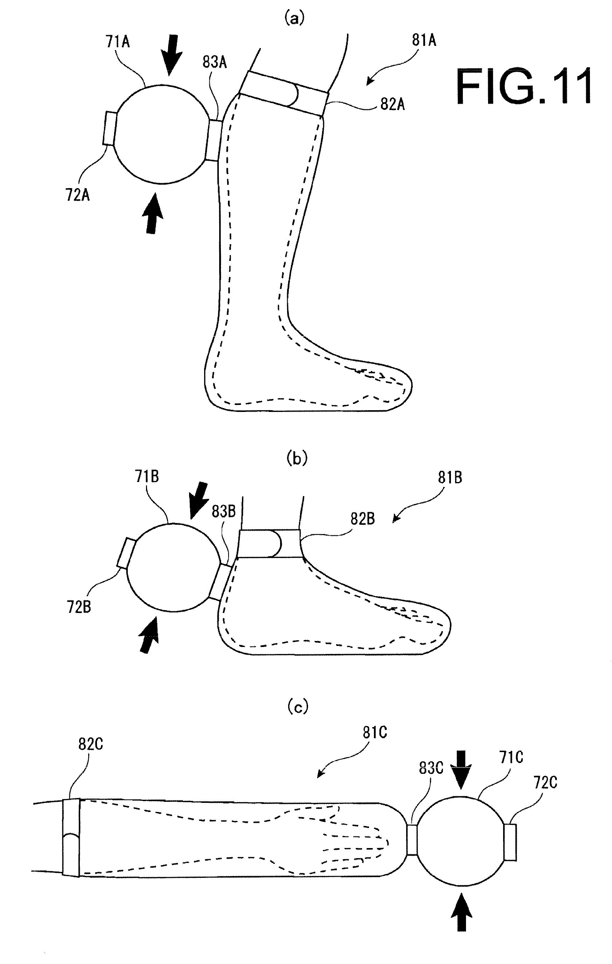

FIG.s 11 and 12 show examples of the living-body pressure bath cover 81 and the pressurizing part 71 connected thereto. FIG. 11(a) is a living-body pressure bath cover 81A for one-side lower extremity (lower part under a knee) of the living body. The living-body pressure bath cover 81A has a stopper 82A at its opening part and a supply portion 83A for introducing inside the gas mist and the gas. The supply portion 83A is connected to the pressurizing part 71, and the gas mist and the gas are supplied into the living-body pressure bath cover 81A through a supply portion 72A of a pressurizing part 71A. FIG. 11 (b) is a living-body pressure bath cover 81B for a foot of the living body. The living-body pressure bath cover 81B has a stopper 82B and a supply portion 83B for introducing the gas mist and the gas into the inside thereof. The supply portion 83B is connected to a pressurizing part 71B, and the gas mist and the gas are supplied into the living-body pressure bath cover 81B through a supply portion 72B of a pressurizing part 71B. FIG. 11(c) is a living-body pressure bath cover 81C for an arm of the living body. The living-body pressure bath cover 81C has a stopper 82C at its opening part and a supply portion 83C for introducing the gas mist and the gas into the inside thereof. The supply portion 83C is connected to a pressurizing part 71C, and the gas mist and the gas are supplied into the living-body pressure bath cover 81C through a supply portion 72C of a pressurizing part 71C.

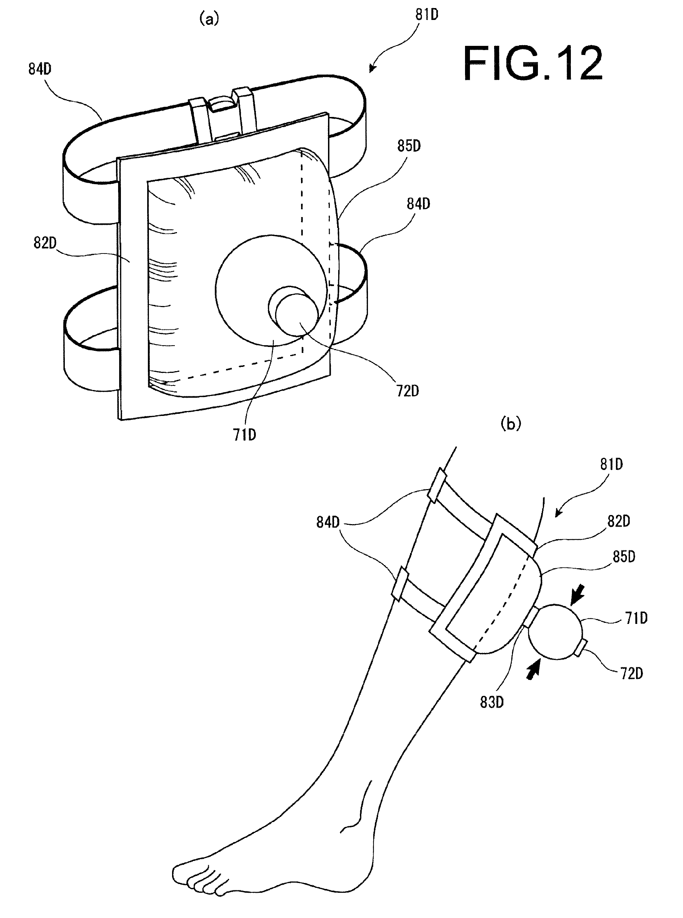

FIG. 12 shows a patch shaped living-body pressure bath covers 81D. FIG. 12(a) is a view showing an outline of the patch shaped living-body pressure bath covers 81D. FIG. 12 (b) is a view showing an external appearance when attaching the patch shaped living-body pressure bath covers 81D to the living body (herein, lower extremity of the living body). The living-body pressure bath covers 81D is composed of a cover part 85D for covering the skin and mucous membrane of the living body, a stopper 82D provided at the margin of the cover part 85D and directly attached to the skin and mucous membrane of the living body, a supply portion 83D for supplying the gas mist and the gas into a space defined by the cover part 85D and the stopper 82D, and fasteners 84D made of belts or strings for fastening the cover part 85D to the living body. The supply portion 83D is connected to a pressurizing part 71D, and through a supply portion 72D of a pressurizing part 71D, the gas mist and the gas are supplied into the living-body pressure bath cover 81D.

An exhaust portion may be formed for exhausting the gas mist and the gas from the inside of the living-body pressure bath cover 81. In addition, the invention may be applied not only to the human living body but to animals.

In the above embodiment, the pressurizing part 71 is the hollow gas storage connected to the living-body pressure bath cover 81, and so far as materials of easily pressurizing as crushing externally the living-body pressure bath cover 81 itself, any materials are sufficient.

### [Third Embodiment]

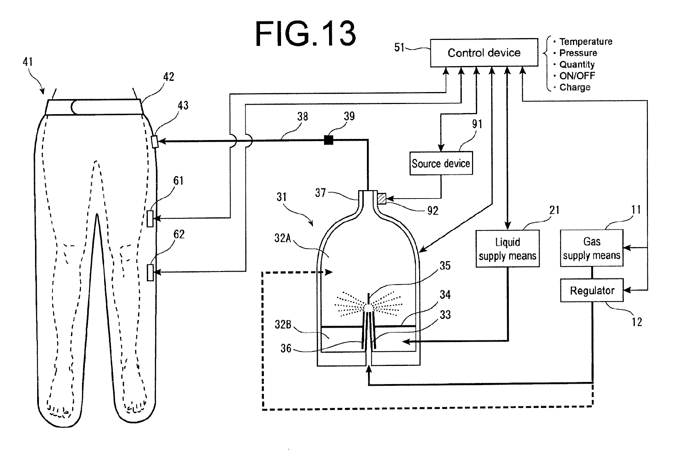

FIG. 13 is the whole schematic view of the gas mist pressure bath system depending on the third embodiment of this invention. This embodiment will explain the gas mist pressure bath system further having a means for charging generated mist. As to the same parts as those of the first embodiment shown in FIG. 1, the same numerals will be given, and detailed explanation will be omitted.

As shown in FIG. 13, the gas mist pressure bath system of this embodiment is arranged with an electrode 92 at the gas mist exhaust portion 37 of the gas mist supply device 31. The electrode 92 is connected to a source device 91, and the control device 51 sets voltage values and performs on-off control.

The electrode 92 supplies an electric charge (minus charge is desirable) when exhausting the mist generated by the gas mist supply device 31 from the gas mist exhaust portion 37. Thereby, the mist is made turn out charged so that adhesion to a charged material can be heightened. For example, if adhesion to the skin and the mucous membrane of the living body, an effect of increasing absorption of the gas by the mist is further heightened, and if the mist contains the above mentioned medicines, penetration into the skin and the mucous membrane can be accelerated.

For carrying out the gas mist pressure bath by using the gas mist pressure bath system of the present embodiment, the living-body pressure bath cover 41 is secured to the living body (herein, as the example, the lower extremity) and closed. In the liquid storing part 32B of the gas mist supply device 31, a liquid of a predetermined amount is in advance poured, and next, the gas is supplied from the gas supply means 11 to the nozzle 33, thereby to generate the gas mist. Then, the control device 51 controls the gas supplying pressure, amount, or the liquid supplying amount, temperature and others. The control device 51 turns on the source device 91, and supplies electric charge from an electrode 92 to the mist. The generated gas mist is supplied from the supply portion 43 into the living-body pressure bath cover 41. When the mist is enough filled in the living-body pressure bath cover 41, the gas is not supplied into the nozzle 33, but directly supplied into the gas mist storing part 32A. When the inside of the living-body pressure bath cover 41 becomes an optimum pressurized and heated condition (around 1.02 to 2.5 air pressure and around 38°C) in view of the measuring values of the manometer 61 and the thermometer 62, the control device 51 once stops supply of the gas mist or the gas and under this condition the gas mist pressure bath is carried out.

### [Fourth Embodiment]

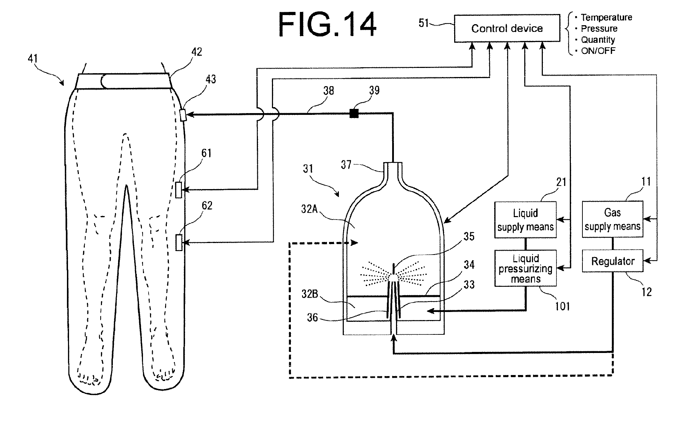

FIG. 14 is the whole schematic view of the gas mist pressure bath system depending on the fourth embodiment of this invention. This embodiment will explain the gas mist pressure bath system further having a liquid pressurizing means for pressurizing the liquid from the liquid supply means and sending it to the gas mist supply means. As to the same parts as those of the first embodiment shown in FIG. 1, the same numerals will be given, and detailed explanation will be omitted.

As shown in FIG. 14, the gas mist pressure bath system of this embodiment is provided with the liquid pressurizing means 101 for pressurizing the liquid and sending it to the gas mist supply device 31. The liquid pressurizing means 101 is composed of a pump for pressurizing the liquid from the liquid supply means 21 and sending it to the liquid storing part 32B of the gas mist supply device 31. By pressurizing the liquid, it makes easy to pressurize and supply of the gas mist of the gas mist supply device 31. Adjustment of supplying pressure in the liquid pressurizing means 101 is carried out by the control device 51.

For carrying out the gas mist pressure bath by using the gas mist pressure bath system of the present embodiment, the living-body pressure bath cover 41 is secured to the living body (herein, as the example, the lower extremity) and closed. In the liquid storing part 32B of the gas mist supply device 31, a liquid of a predetermined amount is in advance poured via the liquid pressurizing means 101. While continuing to supply under pressure the liquid from the liquid pressurizing means 101, the gas is supplied from the gas supply means 11 to the liquid storing part 32B and the nozzle 33, thereby to generate the gas mist. Then, the control device 51 controls the liquid and gas supplying pressure, amount, or the liquid supplying amount, temperature and others. The control device 51 turns on the source device 91, and supplies electric charge from an electrode 92 to the mist. The generated gas mist is supplied from the supply portion 43 into the living-body pressure bath cover 41. When the mist is enough filled in the living-body pressure bath cover 41, the gas is not supplied into the nozzle 33, but directly supplied into the gas mist storing part 32A. When the inside of the living-body pressure bath cover 41 becomes an optimum pressurized and heated condition (around 1.02 to 2.5 air pressure and around 38°C) in view of the measuring values of the manometer 61 and the thermometer 62, the control device 51 once stops supply of the gas mist or the gas and under this condition the gas mist pressure bath is carried out.

The above mentioned explanation has been made with the example of the lower extremities of the living body, and the invention is applicable to various parts. Then, the optimum gas mist pressure bath is performed using the living-body pressure bath cover 41 meeting object parts of the living body.

With the structure as mentioned above, according to the gas mist pressure bath system, it is possible to control the amount, pressure and other of the gas mist within the living-body pressure bath cover by the control device, and so the gas mist pressure bath can be always carried out under the optimum condition.

Since it is possible to generate the gas mist by the very simple structure, reduction of cost of the device can be realized.

Further, pressurization into the living-body pressure bath cover is easy, the gas skin-pass absorption can be more efficiently performed.

The above explanation has been made to the embodiments of the invention, but the invention is not limited to such embodiments, and so far as not deviating from the subject matter of the invention, various kinds of embodiments are, of course, available.

### Industrial Applicability

Thus, the present invention relates to the gas mist pressure bath device, in which the gas mist is prepared by pulverizing and dissolving carbon dioxide and oxygen or the mixed gas of carbon dioxide and oxygen, and the gas mist is directly contacted to the skin or mucous membrane of the living body for improving the blood circulation of the living body, and has an industrial applicability.

### Description of Symbols

11: Gas supply means
12: Regulator
21: Liquid supply means
31: Gas mist supply device
31A, 31B: Plate
32: Storage
32A: Gas mist storing part
32B: Liquid storing part
33: Nozzle
33A: Front end opening of the nozzle
34: Shielding plate
35: Baffle
36: Suction pipe forming member
36A: Sucking pipe
36B: Front end of the sucking pipe
37: Gas mist exhaust portion
38: Gas mist supply pipe
38A: Cornice shaped pipe
39: Liquid drop removing filter
41, 41A, 41B, 41C, 41D, 41E, 41F, 81, 81A, 81B, 81C, 81D: Living-body pressure bath cover
42, 42A, 42B, 42C, 42D, 42E, 42F, 44A, 82, 82A, 82B, 82C, 82D: Stopper
43, 43A, 43B, 43C, 43D, 4E, 43F, 83, 83A, 83B, 83C, 83D: Supply portion
44F: Fastener
45F: Cover part
51: Control device
61: Manometer
62: Thermometer
71, 71A, 71B, 71C, 71D: Pressurizing part
72, 72A, 72B, 72C, 72D: Supply portion
84D: Fastener
85D: Cover part
91: Source device
92: Electrode
101: Liquid pressurizing means

## Claims

1. A gas mist pressure bath system, in which a mist (called as "gas mist" hereafter) is prepared at a density of not less than a predetermined value by pulverizing and dissolving carbon dioxide or oxygen or a mixed gas (called as "gas" hereafter) of carbon dioxide and oxygen and liquid, and the thus prepared gas mist is directly contacted to a skin and mucous membrane of a living body, comprises
a gas supply means,
a gas mist supply means for generating and supplying the gas mist by pulverizing and dissolving the gas and the liquid supplied from the gas supply means, and
a living-body cover member for covering the skin and mucous membrane of the living body and forming a space of sealing inside the gas mist supplied from the gas mist supply means,
wherein the gas mist supply means comprises a liquid storage of storing liquid, a gas mist storage of storing a gas mist, a nozzle part of jetting gas from its front end, a suction pipe of sucking up the liquid stored in the liquid storage until the nozzle front end, and a baffle member provided opposite the nozzle front end, and is **characterized in that** the gas jetted from the nozzle part causes the liquid sucked up by the suction pipe until the nozzle front end to collide with the baffle member and turn out the gas mist by pulverizing and dissolving.

2. A gas mist pressure bath system as set forth in claim 1, further provided with a sensor for measuring supplying conditions of the gas, liquid and gas mist, and
a control means for controlling supplies of the gas, liquid and gas mist based on the measuring values of the sensor.

3. A gas mist pressure bath system as set forth in claim 1 or 2, further provided with a pressurizing means for pressurizing the living-body cover member.

4. A gas mist pressure bath system as set forth in claim 2, wherein the control means supplies the gas mist intermittently into the living-body cover member to perform interval pressurization on the living-body cover member.

5. A gas mist pressure bath system as set forth in claim 3 wherein the pressurizing means pressurizes the living-body cover member intermittently to perform the interval pressurization on the living-body cover member.

6. A gas mist pressure bath system as set forth in claim 1, further provided with a liquid supply means for supplying liquid to the liquid storing part.

7. A gas mist pressure bath system as set forth in claim 6, further provided with a liquid pressurizing means of pressurizing the liquid from the liquid supply means and supplying it to the gas mist supply means.

8. A gas mist pressure bath system as set forth in any one of claim 1, wherein the above mentioned liquid is any one or plural combination of water, ionic water, physiological salt solution, ozone water, purified water or sterilized water.

9. A gas mist pressure bath system as set forth in claim 7, wherein the above mentioned liquid further contains any one or plural combination of menthol, vitamin E, vitamin C derivative, retinol, anesthetic, cyclodextrin, photocatalyst, complex of photocatalyst and apatite, hyaluronic acid, coenzyme Q10, seed oil, propolith, ethanol, gluconic acid chlorohexizine, amphoteric surface active agent, benzalkonium chloride, alkyldiamino ether glycin acetate, sodium hypochlorite, acetyl hydroperaxide, sodium sesquicarbonate, silica, povidone-iodine, sodium hydrogen carbonate, high density carbonate spring, anti-allergic agent, anti-inflammatory agent, anti-febrile, anti-fungus agent, anti-influenza virus, carcinostatic substance, anti-hyper tensive agent, cosmetic agent, or trichogen.

10. A gas mist pressure bath system as set forth in claim 7 or 9, wherein the liquid is supplied into the gas mist supply means under a condition of being heated.

11. A gas mist pressure bath system as set forth in claim 1, wherein sizes of the gas mist supplied from the gas mist supply means to the living-body cover member are not more than 10 µm.

12. A gas mist pressure bath system as set forth in claim 2, wherein the control means holds pressure at 1.02 to 2. 5 air pressure in the living-body cover member when taking the gas mist bath.

13. A gas mist pressure bath system as set forth in claim 1, wherein there is further provided an electric charge supply means for supplying charge to the mist from the gas mist supply means.

14. A gas mist pressure bath system as set forth in claim 13, wherein a charge is minus.

15. A gas mist pressure bath system as set forth in claim 1, wherein the gas mist supply means has a gas mist supply pipe for supplying the gas mist into the living-body cover member, and
the gas mist supply pipe has a filter for removing liquid drops attached to a pipe inside.

16. A gas mist pressure bath system as set forth in claim 1, wherein the gas mist supply means has a gas mist supply pipe for supplying the gas mist into the living-body cover member, and
a whole or one of the gas mist supply pipe is composed of a cornice shaped pipe.

17. A gas mist pressure bath system as set forth in claim 1, wherein the gas mist supply means has a gas mist supply pipe, and
the gas mist supply pipe is provided with a check valve.

18. A gas mist pressure bath system as set forth in claim 1, wherein the living-body cover member is provided at its gas mist supply portion with the check valve.

19. A gas mist pressure bath system as set forth in claim 1, wherein a storage is shaped in dome of convex having inside a curved face toward an upper portion and is formed with a gas mist exhaust portion at the dome shaped top.

20. A gas mist pressure bath system as set forth in claim 1, wherein the gas mist supply means has inside one or plural sheets of pored plates for refining the gas mist.

21. A gas mist pressure bath system as set forth in claim 2, wherein the control means stops the gas from the gas supply means when the pressurizing value within the living-body cover member is higher than the predetermined value.
